# EUROPEAN PATENT APPLICATION

(11) **EP 2 735 303 A1**
(43) Date of publication of application: **28.05.2014**
(21) Application number: 12306459.4
(22) Date of filing: 23.11.2012
(51) Int. Cl.: A61K 8/64, A61Q 7/00, A61Q 7/02, A61P 17/14, A61K 8/41, A61K 8/44, A61K 8/49, C07K 16/18

(54) **Hair growth compositions and methods**

(71) Applicant: Pilosciences, 75008 Paris (FR)
(72) Inventor: Blanchard, Dominique, 44300 Nantes (FR); Pouletty, Philippe, 75005 Paris (FR); De Francisco, Angelita, 75007 Paris (FR)
(74) Representative: Becker, Philippe

(57) **Abstract**

The present invention relates to hair modifying compositions and methods, as well as to compositions and methods for modifying hair growth. The invention more specifically relates to targeted approaches using antibodies or agonists thereof for altering or stimulating hair growth, structure or number. The invention also relates to compositions, such as cosmetic compositions, comprising antibodies and/or further active agents, and their use to modify hair growth and/or to remove unwanted hair. The invention may be used in any mammalian subject, optionally in combination with any other depilation or hair stimulating method or any other hair treatment.

## Description

The present invention relates to hair growth compositions and methods, as well as to compositions and methods for modifying hair growth, structure or number. The invention more specifically relates to targeted approaches using antibodies for modifying hair growth, structure, or number. The invention also relates to compositions, such as cosmetic compositions, comprising antibodies and/or further active agents, and their use to modify hair growth and/or to remove unwanted hair. The invention may be used in any mammalian subject, optionally in combination with any other hair depilation or hair stimulation product, method or any other hair treatment.

### INTRODUCTION

A number of methods are currently used to remove unwanted hair or to stimulate hair growth. They include mechanical techniques such as shaving or waxing, physical techniques such as electrolysis or laser treatment, as well as chemical techniques such as depilatory creams or lotions. These methods, however, have drawbacks such as a poor efficacy, side effects, or discomfort and pain.

A more limited number of methods have also been developed for hair growth reduction, such as the use of inhibitors of certain enzymes, which have been reported as controlling the rate of hair growth. Such enzymes include, for instance, elastase (EP943311), deoxyhypusine synthetase or deoxyhypusine hydroxylase (US6,060,471), 5-alpha reductase, ornithine decarboxylase (US4,720,489), or S-adenosylmethionine decarboxylase. Various chemical inhibitors of such enzymes have been disclosed in the art. In particular, chemical inhibitors of ornithine decarboxylase have been disclosed and eflornithine (α-difluoromethylornithine or DFMO), an ornithine analogue that competes for ornithine decarboxylase is currently approved by the FDA and the European commission in the treatment of facial hirsutism (excessive hair growth).

As indicated above, these chemical inhibitors may have drawbacks such as a poor efficacy or side effects. In particular, these drugs may not be specific enough, or they may, upon topical application, enter the general blood circulation and distribute in the entire body. Furthermore, these agents often have a short stability of half-life and exert a limited effect.

Accordingly, there is a need for alternative compositions and methods for modifying (e.g. retarding or stimulating) hair growth, which are easy to use, efficient, devoid of side effects, and which may be used alone or in combination with existing methods or agents.

### SUMMARY OF THE INVENTION

The invention provides improved compositions and methods for modifying hair growth.

More specifically, an object of the invention resides in a method for modifying hair growth in a subject, comprising providing to the subject a composition comprising an antibody, or an agonist thereof, that binds a hair growth molecule.

A further object of the invention relates to a composition comprising an antibody, or an agonist thereof, that binds a hair growth molecule, for use in modifying hair growth in a subject.

A further object of the invention relates to a composition comprising an antibody, or an agonist thereof, that inhibits a hair growth molecule, for use in retarding hair growth in a subj ect.

Another object of the invention resides in a method for retarding hair growth in a subject, comprising providing to the subject a composition comprising an antibody, or an agonist thereof, that inhibits a hair growth molecule.

As will be disclosed in further details, the hair growth molecule is preferably a protein of the polyamine pathway or a hair follicle protein. Preferred examples of such hair growth molecules include, more preferably, ornithine decarboxylase (ODC), spermidine, spermine, spermidine synthase, spermine synthase, keratins (such as K16), keratin intermediate filament proteins or keratin filaments associated proteins, such as trichohyalin or filaggrin.

Furthermore, in preferred embodiments, the composition comprises a combination of at least two antibodies that react with the same or a distinct hair growth molecule.

In a particular embodiment, the composition further comprises an enhancer and/or one or several acceptable excipients or carriers.

A further object of the invention also resides in a method for modifying hair growth in a subject, comprising providing the subject a composition comprising (i) an antibody, or an agonist thereof, that binds (preferably that inhibits an activity of) a hair growth molecule, (ii) an enhancer and (iii) an excipient or a carrier.

Another object of the invention resides in a composition comprising (i) an antibody, or an agonist thereof, that binds (preferably that inhibits) a hair growth molecule, (ii) an enhancer, and, optionally, (iii) a suitable excipient or carrier.

The enhancer is preferably a compound that alters or modulates hair growth or structure and/or that enhances the activity of the antibody, preferably its delivery into the hair follicle. The enhancer may be a depilatory agent, an anti-angiogenic agent, or an anti-inflammatory agent.

Another object of invention is a composition comprising a trichohyalin inhibitor, for use to alter hair growth.

Another object of invention is a composition comprising a keratin 16 (K16) inhibitor, for use to alter hair growth.

A further object of the invention is a composition comprising a spermine synthase inhibitor or a spermidine synthase inhibitor, for use to alter hair growth.

Still a further object of the invention is a composition comprising an anti-ODC antibody for use to alter hair growth.

The compositions of the invention are preferably provided to the subject by dermal application, such as by topical application on an area of the skin of the subject, or transdermally, by injection in the skin of the subject. Furthermore, the compositions may be applied repeatedly, either without any other treatment or in combination with (e.g., before, simultaneously, or after) chemical, mechanical or physical depilation. In a preferred embodiment, the compositions are formulated as a cream, gel, paste, oil, emulsion, including microemulsion or nanoemulsion, nanoparticles, liposome, or spray, and/or are applied with a device or formulated in a patch device or a technical textile.

Preferred compositions of the invention are cosmetic compositions, such as creams, oils, gels, soaps, sprays, etc, and/or are applied topically.

The invention may be used in any mammalian subject in need thereof, typically any human subject and is suitable to alter the growth of any body hair. The invention is particularly suited to retard hair growth.

### LEGEND TO THE FIGURES

Figure 1: Schematic representation of the polyamine pathway.
Figure 2: HGP proteins identified on Coomassie blue stained gel.
Figure 3: Biochemical characterization of an anti-HGP polyclonal antibody by Western blot.
Figure 4: Alteration of hair follicle structure following application of anti-HGM antibodies. (A) Upper part of the follicle, near the infundibulum ; A14: anti-ODC antibody. (B) Sebaceous gland region ; A14: anti-ODC antibody.

### DETAILED DESCRIPTION OF THE INVENTION

The invention provides improved compositions and methods for modifying (e.g., decreasing, retarding or stimulating) hair growth.

The invention, in one aspect, is based on the use of antibodies directed against hair growth molecules. The invention shows that antibodies can reach the target site (e.g., hair follicle) and can modify, temporarily or permanently, the structure or growth of hair or of any of the hair follicle components, resulting in a retardation or alteration or stimulation of hair growth and/or hair number. The invention also discloses, for the first time, that topical antibodies are effective at the hair follicle level to retard hair growth, and that antibodies can use the transfollicular route to specifically bind to hair follicle proteins, without entering the blood circulation.

The invention also stems from the identification of particular target proteins that may be inhibited to substantially alter hair growth.

The invention also discloses novel combination products that can be used to effectively modify hair growth by acting on hair structure, hair growth and/or hair number. The compositions of the invention may be delivered to the targeted site into the hair follicle either through a transdermal pathway or a trans-follicular pathway (both being common topical routes of drug delivery).

### Definitions

Within the context of the present invention, the term "modifying hair growth" includes altering, or retarding or stimulating hair growth.

Within the context of the present invention, the term "retarding hair growth" or "altering hair growth" includes inhibiting, reducing, delaying or slowing down the growth of hair or of hair components, or the number of hair. Retarding may be transient or permanent. The invention is particularly suited for inhibiting, reducing, delaying or slowing down the growth of body hair or of body hair components, or the number of body hair.

Permanent hair reduction is generally defined as the long-term, stable reduction in the number of hairs re-growing after a treatment regime (which may include several sessions). Permanent hair reduction does not necessarily imply the elimination of all hairs in the treatment area.

In a preferred embodiment, retarding hair growth means delaying hair re-growth after treatment.

An agonist of an antibody designates any molecule that binds a similar epitope as the antibody and produces a biological effect of similar nature. Typical agonists are compounds that can compete with the antibody for binding to the target protein. A biological effect of "similar nature" designates the same type of effect (e.g., if the antibody inhibits a protein, the agonist shall also be an inhibitor of said protein), even if the extent of the effect may be different. The agonist may be, for instance, an antibody or fragment or derivative thereof, a chemical compound, a nucleic acid molecule, a peptide, etc.

An "inhibitor" designates any molecule, such as an antibody or an agonist thereof, which can inhibit the activity of a target molecule. Inhibition may occur through direct binding of the inhibitor to the target, or indirectly, by interfering with the synthesis or activation pathway of the target. Inhibition designates any reduction by at least 10%, preferably at least 15, 20, 25 or 30% of the activity of the target molecule.

A "stimulation" of hair growth designates any increase by at least 5%, preferably at least 15, 20, 25 or 30% of hair growth or size or number.

Dermal delivery more specifically includes topical delivery or application (e.g., on the surface of the skin), and intra-dermal delivery (e.g., by intradermal injection).
Topical delivery designates the application of a drug-containing formulation on the surface of the skin. Topical application causes a limited delivery or effect of the drug to the surface of the skin or within the skin itself, preferably within the epidermis. Intradermal injection includes injection in various layers of the skin, such as the dermis, hypodermis and epidermis.
A preferred dermal application is a topical application.

### Hair Growth Molecule

The invention is based on the use of agents, such as antibodies, which alter Hair Growth Molecules. The term "Hair Growth Molecule" ("HGM") designates any molecule, preferably any protein that participates in the formation, viability, or growth of hair and in the hair follicle cycle. Hair Growth Molecules of proteinaceous nature are designated "Hair Growth Proteins" ("HGP"). Hair Growth Molecules may be of two types: positive HGMs, which favor the growth of hair, or negative HGMs, which reduce hair growth. Antibodies that inhibit positive HGMs lead to a retardation of hair growth, while antibodies that inhibit negative HGMs lead to a stimulation of hair growth.

The hair follicle constitutes the pilo-sebaceous unit and its associated structures, the sebaceous gland, the apocrine gland ant the arrector pili muscle. When looking at a cross-section of the hair follicle, its epithelium forms a cylinder with at least eight different concentric layers, each one expressing a distinct pattern of keratins. Starting from the periphery, these layers include:
- The outer root sheat
- The companion layer,
- The inner root sheat (Henle's layer, Huxley's layer and cuticle), and
- The hair shaft (hair cuticle, medulla and cortex). The hair shaft consists of terminally differentiated, dead keratinocytes that are produced by the hair follicle.

In the bulge region, the outer root sheath contains the epithelial hair follicle stem cells. The central part of the hair follicle epithelium holds the hair shaft. The entire hair follicle epithelium is surrounded by a mesoderm-derived connective tissue sheat, a loose accumulation of collagen and stromal cells resting upon a basement membrane. After birth, mature and actively growing hair follicles eventually become anchored in the subcutis, and periodically regenerate by spontaneously undergoing repetitive cycles of growth (anagen), apoptosis-driven regression (catagen) and relative quiescence (telogen). Generation of the new hair shaft depends on the activation of hair-specific epithelial stem cells, harbored in the bulge region of the hair follicle epithelium (Schneider et al 2009).

Within the context of this invention, the term HGP includes structural proteins that form the structure of hair (including proteins of the inner root sheath, the outer root sheet, the follicle matrix and dermal papilla, and the hair shaft), as well as functional proteins that contribute to the formation or elongation of hair and proteins involved in keratinocyte proliferation.

Other HGM include molecules, such as lipids or sugars, which are involved in the hair growth cycle. Examples of such molecules include, without limitation, proteoglycans such as chondroitin sulfate proteoglycans, heparan sulfate proteoglycans, BM-CSPG, Galectin-1 or Versican.

Preferred HGMs are HGP, more preferably proteins of the polyamine pathway and hair follicle proteins. The invention shows such proteins represent effective targets for altering hair growth, especially for effective antibody-mediated inhibition.

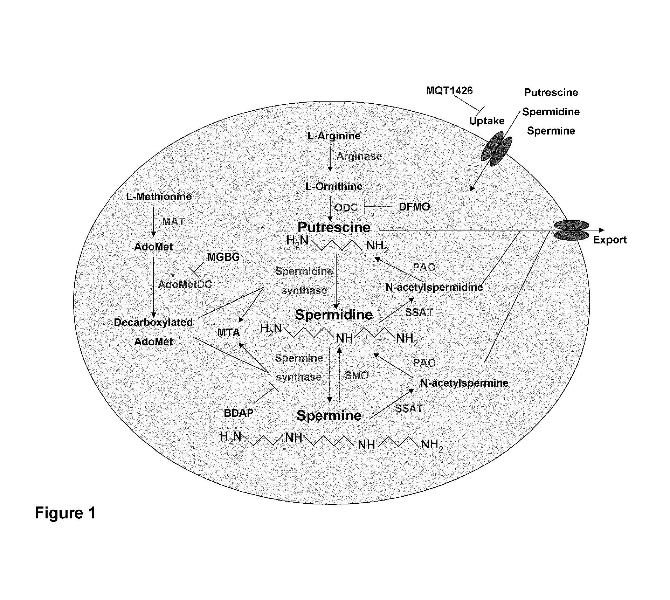

The polyamine pathway is schematically represented on Figure 1. The pathway includes several polyamine proteins, namely putrescin, spermidine and spermine, the relative levels of which are regulated by a number of specific enzymes, ornithine decarboxylase (ODC), spermidine/spermine acetyl-transferase (SSAT), spermidine synthase and spermine synthase in particular.

Ornithine decarboxylase (ODC) generates putrescine and is the first rate-limiting step in polyamine biosynthesis. It has a short half-life, and is regulated at multiple steps. Eflornithine, 2-difluoromethylornithine (DMFO) acts on ODC. Another rate-limiting enzyme in polyamine synthesis can be S-adenosylmethionine decarboxylase (AdoMetDC), the activity of which provides the aminopropyl donor for the synthesis of both spermidine and spermine. Spermine oxidase (SMO) is an inducible oxidase that is involved in the cytotoxic response of cells to specific stimuli through the production of H₂O₂, and N1-acetylpolyamine oxidase (APAO) is a constitutively expressed, peroxisomal oxidase that is rate limited by the availability of N1-acetylated polyamines. Spermidine/spermine N1-acetyltransferase (SSAT) is an inducible enzyme that is crucial for the maintenance of polyamine homeostasis and is also implicated in the cytotoxic activity of several polyamine analogues.

Proteins of the polyamine pathway are involved in various functions, including cell proliferation. Increased levels of polyamines have been identified in tumor cells, and it has been reported that such increased levels arise from an enhanced polyamine synthesis and from a capture of circulating polyamines. Different agents that affect the polyamine biosynthesis are investigated in oncology (Casero et al., Nature Review - Drug Discovery, vol6, May 2007, 373390).

Overall, however, there are limited data in the literature on the impact of these molecules on the hair follicle cycle. In addition, the use of antibodies directed against any of these proteins to regulate hair growth has never been reported.

The present invention shows that certain proteins of the polyamine pathway represent potent target proteins for antibodies, and that antibodies can be used as alternatives to chemical modulators for altering hair growth. Accordingly, in a particular embodiment, a preferred positive HGP of the present invention is a protein of the polyamine pathway which directly or indirectly facilitates or improves or stimulates hair growth. Most preferred examples of such proteins include ODC, spermidine or spermine, which positively regulate hair growth.

A second preferred class of positive HGP for use in the present invention is represented by Hair follicle proteins. Hair follicle proteins are proteins involved in the hair follicle cycle and in hair growth. They include keratins, such as K16, keratin-intermediate-filaments ("KIF" or "IF"), and keratin filaments associated proteins ("KFAP"), such as AHF/trichohyalin and filaggrin.

The term Keratin is often misunderstood to be a single substance, even though it is composed of a complex mixture of proteins, such as keratins (including Keratin Intermediate Filaments which contain depolymerized keratins), Keratin Filaments Associated Proteins (or "KFAP") and enzymes extracted from epithelia (Tomlinson et al. 2004). Keratins are found only in epithelial cells and are characterized by unique physicochemical properties (Steinert et al. 1982; Sun et al. 1983). They are resistant to digestion by the proteases pepsin or trypsin and are insoluble in dilute acids, alkalines, water and organic solvents (Block, 1951; Steinert et al. 1982).

Different types of keratins are distinguished according to various characteristics, such as physicochemical properties, or according to the cells and tissues that produce certain keratins. Keratins in simple, non-stratified epithelia are of different types than those in stratified epithelia. Epithelial cells in simple as well as in stratified epithelia always synthesize particular keratins in a regular basis. These keratins are referred to as the primary keratins of epithelial cells, such as K8/K18 in simple epithelia (Pekny & Lane, 2007) or K5/K14 in stratified epithelia (Moll et al. 1982). These epithelial cells can also produce other keratins in addition to or instead of the primary keratins and these keratins are referred to as secondary keratins, such as K7/K19 in simple epithelia or K15 and K6/K16 in stratified epithelia. Keratins produced in the suprabasal cells of the soft-keratinizing and cornifying epidermis of the skin differ from the keratins produced in the suprabasal epithelial cells of the hard-keratinizing and cornifying epidermis of the hair cortex, hair cuticle or plate of the human fingernail.

K16 has a MW of 46 kDa and an isoelectric pH of 5.1 (Moll et al. 1982). The amino acid sequence of K16 is 82% identical in the human and mouse (Wawersik & Coulombe, 2000). K16 expression defines a subset of epithelial cells during the morphogenesis of the skin and of cells in the companion layer of the outer root sheath of hairs (Bernot et al. 2002; Larouche et al. 2008). K16 (and its basic partner K6) is a marker for the activated state of a keratinocyte, which is able to migrate, produce extracellular matrix and hyperproliferate (Freedberg et al. 2001). K16 can polymerize to form keratin filaments with the basic keratins K5, K6b and K8 (Coulombe et al. 1998). K16 is involved in the hair cycle, and the expression of K16 peaks between the catagen and telogen phases of the hair cycle.

The KFAPs are non-filamentous structural proteins that are produced in keratinocytes of the *Stratum granulosum* of stratified keratinizing and cornifying epithelia and stored in keratohyalin granules (Jessen, 1970). In general, two types of KFAPs can be distinguished based on the structures to which the KFAPs contribute. The KFAPs (e.g. involucrin, loricrin) that are stored in a subset of keratohyalin granules (i.e. the L-granules) form the subcytolemmal cornified cell envelope. The KFAPs that are stored in another subset of keratohyalin granules (i.e. the F-granules) combine with keratin filaments to form the filament-matrix complex stabilizing the cytoskeleton of cornifying keratinocytes (Eckert et al. 2005). Different matrix-forming KFAPs are produced in the soft-keratinizing and cornifying keratinocytes of the interfollicular epidermis (e.g. profilaggrin/filaggrin) compared with those produced in the modified soft-keratinizing and cornifying cells of the hair follicle (e.g. trichohyalin, trichoplein) (Mack et al. 1993; Nishizawa et al. 2005). This corroborates that the soft keratinization and cornification processes in the interfollicular vs. those in the follicular epidermis are different.
The onset of the synthesis of KFAPs and keratins is coordinated. For example, the synthesis of the KFAP profilaggrin' starts shortly after the expression of K1 and K10 (Dale et al. 1985). This profilaggrin has a calcium-binding region in its N-terminal domain and is cleaved by the proteinase caspase-14 into several filaggrin molecules, which then bind to keratins and keratin filaments (Mack et al. 1993; Denecker et al. 2007). The observations so far indicate that profilaggrin/filaggrin (and caspase-14) are produced only in softkeratinizing and -cornifying stratified epithelia (Bragulla & Budras, 1991). Filaggrin itself is a cationic protein (Mack et al. 1993). It is sandwiched between the rod domains of two keratin filaments with which it establishes ionic bonds (see 'ionic zipper model' of Mack et al. 1993) (Bragulla & Homberger 2009).

Trichohyalin (or AHF - Anagenic Hair Follicle antigen) is an abundant protein in the outer root sheet of the lower part of the hair follicle in anagen phase. Its role in the assembling of intermediate keratin filaments has been described (Takase, Hirai, J Dermatol Sci. 2012 Feb; 65(2):141-8*).* The amino acid and nucleic acid sequences of trichohyalin are disclosed, for instance, in US patents US5958752 and US5,616,500, which propose their use in woundcare.

Preferred target hair follicle proteins for the present invention are KFAP, even more preferably trichohyalin or filaggrin. The results contained in the present application show that agents such as antibodies, which inhibit these proteins, can effectively alter hair growth. Surprisingly and remarkably, the invention shows that antibodies applied topically are able to effectively affect hair growth.

### Antibodies

One aspect of the invention is based on the provision (e.g. dermal administration, topical application) of anti-HGM antibodies for modifying hair growth. The present invention, for the first time, discloses the skin application of antibodies and the ability of these antibodies to reach the active site (e.g., hair follicle) where they can exert a retarding or stimulating effect on hair growth. The invention is particularly surprising since this is the first time antibodies are topically applied. The invention is advantageous since antibodies are highly specific and do not affect distinct structures. Also, antibodies may be designed or formulated so as to remain in the epidermis and not to reach the dermis and/or blood circulation, thereby avoiding systemic distribution. Furthermore, antibodies can have long-lasting effects and therefore provide sustained growth retardation effect. In particular, while chemical compounds have short half-lives, between minutes or an hour, antibodies can remain active for several days, thereby further sustaining the growth retardation effect.

The invention therefore relates to the administration of anti-HGM antibodies, or agonists thereof, for modifying hair growth. The antibodies of the invention shall react with (e.g., bind to and, preferably, inhibit an activity of) the hair growth molecule. Such inhibition designates any reduction by at least 10%, preferably at least 15, 20, 25 or 30% of the activity of the target HGM. The inhibition is usually transient, depending on the concentration of antibody applied, or on the duration on treatment, for instance. However, a transient inhibition of such HGM is sufficient to cause a prolonged retardation or stimulation in hair growth. In a particular embodiment, the antibody may inhibit any activity of a target HGP, such as its ability to form a complex with one or several other proteins, its ability to adopt a particular conformation, its ability to bind to a receptor or to another protein, its catalytic activity, etc.

Antibodies of the invention may be polyclonals or monoclonals. Antibodies of the invention also include antibody fragments or derivatives, such as Fab fragments, Fab'2 fragments, CDRs, single chain antibodies, diabodies, tetrabodies, multifunctional antibodies, functionalized, humanized or human antibodies, and the like.

Preferred antibodies of the invention are specific for their target HGM, i.e., they do not bind with high affinity to any antigen other than the target HGM. In the case of monoclonal antibodies, it is preferred to use antibodies that bind to the cognate antigen and essentially do not bind another antigen. Preferably, an antibody binds specifically to an antigen when said binding occurs with at least one log more affinity than to other non-structurally related antigens. The invention may also use polyclonal antibodies directed against either a particular target HGM or against a group of target HGMs. Specificity of such polyclonals may be defined by their mode of production, e.g. the immunization with a target HGP (or group of HGPs) and the removal of antibodies that do not bind the immunogen.

Antibody specificity can be assessed by techniques known per se in the art such as binding assay using immunoblotting and/or immunoprecipitation. Specificity may also be demonstrated by testing, e.g., by flow cytometry analysis, binding of the antibody to living mammalian cells, preferably human cells, which do not express hair follicle proteins. The absence or weak binding to such cells, or the depletion of antibodies which bind to said cells, generates antibody composition with high specificity.

Anti-HGM antibodies can be produced by (i) preparing an HGM immunogen, (ii) administering the immunogen to a selected non-human animal species and (iii) collecting the antibodies or antibody-producing cells. If desirable, in further step(s), the specificity of the antibodies may be tested and/or the selected antibodies may be further purified, the sequences of the antibody may be determined and used to produce recombinant antibodies or antibody fragments or derivatives.

An immunogen may be prepared by techniques known per se in the art. To produce anti-HGM antibody, the immunogen may be the entire HGM or an epitope-containing fragment thereof. The HGM or fragment may be in pure form or not, and may be free or associated to a carrier. To generate antibodies against HGM, the present invention discloses a method where a hair follicle is digested and the proteins partially purified. The proteins may further be fragmented to obtain particular epitopes thereof. The immunogen is then typically formulated with an adjuvant to increase the immune response. Administration of the immunogen is generally by injection, which may be repeated (e.g., four to five injections). Antibodies may be prepared in various animal species; e.g., small animals such as rodents (mouse, rat, hamster), larger mammals (rabbit, goat, sheep, donkey, horses), or poultries. Polyclonal antibodies or antibody producing cells (e.g., B lymphocytes) may be collected from the blood of the immunized animals, which may be sacrificed. Monoclonal antibodies are easily obtained from lymphocytes isolated from the immunized animals using techniques well known per se in the art (see e.g., *Kolher and Milstein*). Rabbit monoclonal antibodies can be prepared using appropriate technology (e.g. Epitomics). Antibody fragments (Fab'2 or Fab) may be produced by specific enzymatic cleavages, papain or pepsin, and purified according to commonly used protocols. ScFv can be produced by techniques well known in the art.

The specificity of the antibodies or agonists thereof may be tested/ensured by various techniques, e.g., by removing antibodies which do not bind the immunogen and/or by verifying cross reactivity with other antigens and/or by measuring the affinity of the antibodies for the cognate antigen. In this regard, antibodies may be adsorbed on tissues (e.g. skin explants), cells (e.g., epithelial cells), or immobilized on gel adsorbants prepared with a purified HGP, to remove non-specific antibodies directed against a crude protein extract.

Preferred antibodies of the invention are polyclonal antibodies obtainable by immunization of a non-human mammal with an immunogenic composition comprising a HGP or a fragment thereof, which antibodies bind said immunogenic composition and essentially do not bind the surface of mammalian cells lacking hair follicle proteins.

Other preferred antibodies of the invention are monoclonal antibodies that specifically bind a HGP protein.
The production of anti-HGP antibodies following the above procedure is illustrated in the experimental section. Furthermore, antibodies against some of the target proteins selected by the inventors are commercially available, and have been developed for different purposes. Examples of such antibodies are listed in table II in the experimental section.

In the case of Trichohyalin, most preferred antibodies for use in the present invention are antibodies which bind to trichohyalin and inhibit the interaction between trichohyalin and the keratin meshwork. Trichohyalin latches onto the keratin bundles by its C-terminus and rearranges the keratin meshwork by intrinsic cohesive activity for the granule formation. Most preferred antibodies are antibodies that bind an epitope comprised in the C-terminal part of the protein, more specifically within the first 15 C-ter amino acid residues.

Antibodies against ODC may be generated against the entire protein. Preferred antibodies inhibit at least one enzymatic activity of ODC such as the ability to produce putrescin. Antibodies may be generated against the binding or catalytic site. Particular antibodies for use in the present invention bind an epitope comprised within amino acids 180-433 of human ODC.

Antibodies against spermine synthase may be generated against the entire protein. Preferred antibodies inhibit at least one enzymatic activity of spermine synthase such as the ability to produce spermine. Antibodies may be generated against the binding or catalytic site. Particular antibodies for use in the present invention bind an epitope comprised within amino acids 1-229 or 144-336 of human spermine synthase.

Other preferred agents for use in the invention are agonists of the above antibodies. Agonists may be produced or tested in any competition binding assay between the selected antibody and the target. As indicated above, the agonist may be any molecule that binds the same or a similar epitope as the antibody and inhibits the target protein. Examples of agonists include antibodies and fragments and derivatives thereof, as well as other polypeptides or peptides, nucleic acids (e.g., aptamers), or small molecules.

In another particular embodiment, the antibody for use in the present invention is a catalytic antibody, particularly a catalytic antibody having keratinase activity.

In another particular embodiment, the antibody for use in the present invention is an antibody that stimulates hair growth. A particular example is an antibody, or an agonist thereof, that binds SSAT (spermidine-spermine acetyl-transferase). By inhibiting SSAT, the antibody causes an increase in the pool of spermidine and spermine, while the pool of putrescin decreases, which favors hair growth.

In a preferred embodiment, the composition of the invention comprises an antibody, or an agonist thereof, that binds an HGP of the polyamine pathway, even more preferably an antibody, or an agonist thereof, that binds a protein selected from ODC, spermidine synthase, spermine synthase, spermidine, or spermine.

In another preferred embodiment, the composition of the invention comprises an antibody, or an agonist thereof, that binds a protein of the hair follicle, more preferably an antibody, or an agonist thereof, that binds a keratin, KIF or KFAP protein, preferably selected from keratin K16, trichohyalin, or filaggrin.

Most preferred compositions of the invention comprise an anti-trichohyalin antibody or an agonist thereof, an anti-filaggrin antibody or an agonist thereof, or an anti keratin K16 antibody or an agonist thereof.

The compositions of the invention may comprise only one antibody or agonist against one HGP, or several antibodies or agonists against distinct epitopes of the same HGP, or several antibodies or agonists that bind distinct HGPs. In this regard, a particular composition of the invention comprises:
- an anti-hair follicle protein antibody, or an agonist thereof, preferably an anti-trichohyalin antibody, an anti-filaggrin antibody, or an anti keratin k16 antibody, and
- an anti-polyamine protein pathway antibody, or an agonist thereof, preferably an antibody that binds ODC or spermidine.

In the compositions of the invention, each antibody or agonist is generally present at a dosage of from about 1 µg/mL to about 50 mg/mL, typically from about 0.1 mg/mL to about 50 mg/mL.

### Combinations

As indicated above, the compositions preferably comprise one or several further active agents.

The active agent may be of various natures, such as typically a protein, a peptide, or a small compound. The active agent may be any agent that alters hair growth or structure, and/or that enhances or facilitates the activity of the anti-HGM antibody.

In this regard, in a preferred embodiment, the invention relates to compositions and methods that combine an anti-HGM antibody, or an agonist thereof, and an enhancer.

An object of the invention therefore resides in a method for modifying hair growth in a subject, comprising providing a subject with a composition comprising (i) an antibody, or an agonist thereof, that binds a HGM, preferably that inhibits an activity of a hair growth protein, (ii) an enhancer and (iii) an excipient or carrier.

Another object of the invention resides in a composition comprising (i) an antibody, or an agonist thereof, that binds a HGM, preferably that inhibits a hair growth protein, (ii) an enhancer, and, optionally, (iii) a suitable excipient or carrier.

The enhancer is preferably a compound that alters or modulates hair growth or structure and/or that enhances the activity of the antibody, preferably its delivery into the hair follicle.

The enhancer may be a depilatory agent, an anti-angiogenic agent, or an anti-inflammatory agent. In a particular embodiment, the enhancer is a small compound that alters hair growth or structure. Such compounds include chemical modulators of the polyamine pathway, particularly chemical inhibitors of ODC. Preferred examples of such agents are listed in the table I below.

**Table I**

| **Molecule** | **Structure** | **Target** | **Ref** |
|---|---|---|---|
| N-(4'-Pyridoxyl)-Ornithine(BOC)-Ome **(POB)** | 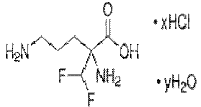 | Ornithine Decarboxylase (ODC) | Merck Biosciences (497985) |
| 4-amidinoindan-1-one-2'-amidinhydrazone **(SAM486A/CGP-48664)** | 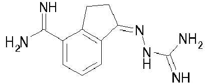 | AdoMetDC | **BOC Sciences** 45-16 Ramsey Road Shirley, NY 11967, USA http://www.bocsci.com/ (Ref 149400-88-4) |
| S-adenosyl-1,12-diamino-3-thio-9-azadodecane (**AdoDATAD**) | 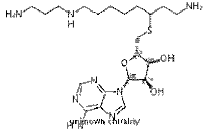 | Spermine Synthase | Hangzhou Sage Chemical Ltd www.sagechem.com |
| N1,N11-Diethylnorspermine tetrahydrochloride **(BENSpm = DENSpm)** | 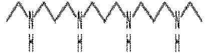 | SSAT & SMO | Tocris http://www.tocris.com/ |

In this regard, an object of the invention also resides in the use of any one of the above 4 compounds for retarding hair growth.

The above small compounds may be used in the compositions of the invention at a dosage of from about 1 to about 10% w/w of the total weight of the composition.

Further examples of small drug enhancers include known depilatory agents such as thioglycolate (e.g.; potassium thioglycolate, calcium thioglycolate, ammonium thioglycolate, glyceryl monothioglycolate, monoethanolamine thioglycolate, diammonium dithiodiglycolate), dithioerythritol, thioglycerol, thioglycol, thioxanthine, thiosalicylcic acid, N-acetyl-L-cysteine, lioic acid, NaHSO3, LiS, Na2S, K2S, MgS, CaS, SrS, BaS, (NH4) 2S, sodium dihydrolipoate 6,8-dithiooctanoate, sodium 6,8-dithiooctanoate, salts of hydrogen sulphide for example NaSH or KSH, thioglycolic acid, 2-mercaptopropionic acid, 3-mercaptopropionic acid, thiomailic acid, ammonium thiolactate, thioglycolamide, homocysteine, cysteine, glutathione, dithiothreitol, dihydrolipoic acid, 1,3-dithiopropanol, thioglycolamide, glycerylmonothioglycolate, thioglycolhydrazide, hydrazine sulphate, hydrazine disulphate, triisocyanate, guanidine thioglycolate, and/or cysteamine.

Further examples of depilatory agents include t-butyl-hydroquinone, bisulphite and/or sulphite compound selected from bisulphites or sulphites of potassium, calcium, lithium, barium or strontium; bisulphite or sulphite alkaline salts with a strong organic base ; and bisulphitic aldehyde or ketone compounds.

A preferred class of depilatory agents for use in combination with an antibody of the invention is thioglycolate.

Another particular class of depilatory agents for use in combination with an antibody of the invention is an anti-elastase. Examples of such compounds have been disclosed, e.g., in US 2005/0147631.

In another embodiment, the enhancer is an enzyme that alters hair growth or structure. Preferred examples of such enzymes include proteases, such as a keratinase, in particular KerA (*Bacillus subtilis*). Such enzymes alter the structure of hair. In addition, they can facilitate delivery of antibodies to the hair follicle by altering the integrity of the superficial layer of skin, in particular of the stratum corneum. Keratinases are disclosed in the art and have already been used in depilation/shaving creams (see e.g., WO2012/048334 ; WO2004/033668 or US2005281773). They have, however, never been combined to antibodies in order to combine their effects and potentiate the activity of antibodies.

Keratinases may be used in the compositions of the invention at a dosage of from about 0.5 to 50 mg/mL.

In this regard, in a particular embodiment, the invention relates to a composition comprising :
(i) an antibody that inhibits a hair growth protein, or an agonist thereof,
(ii) an enhancer, and
(iii) a topical excipient or a cosmetically acceptable vehicle.

The enhancer agent is preferably a keratinase, which facilitates access of an antibody to the hair follicle, or a chemical depilatory compound (such as a thioglycolate compound), or anti-angiogenic agent, an anti-inflammatory agent, an anti-elastase, or a 5-alpha-reductase inhibitor.

A further object of the invention resides in a composition comprising:
(i) an antibody that inhibits a hair growth protein, preferably a KFAP protein, or an agonist of said antibody; and
(ii) a keratinase and/or
(iii) a chemical inhibitor of an enzyme of the polyamine pathway, preferably of ODC.

When the antibody or agonist thereof stimulates hair growth, the enhancer may be a further agent that stimulates hair growth. Examples of such agents include, for instance, analogs of nitric oxide and inhibitors of 5α-reductase. Analogs of nitric oxide stimulate guanylate cyclase, causing a vasodilatation effect. 5α-reductase inhibitors allow the transformation of testosterone into di-hydro-testosterone (DHT), which favors hair growth. Examples of such inhibitors include Dutasteride and finasteride.

A further object of the invention resides in a topical composition comprising an antibody that inhibits a hair growth molecule, or an agonist thereof, and a topical excipient.

A further object of the invention resides in a topical composition comprising an antibody that stimulates a hair growth molecule, or an agonist thereof, and a topical excipient.

The above product combinations exhibit substantial advantages. In particular, the combination potentiates the effect of each compound resulting in a synergistic effect. The combination also allows a reduction in the unitary dosages which decreases the toxicity. The compositions of the invention also exhibit specific effect due to the antigen-specificity of the antibodies.

### Formulation

The compositions and methods of the invention are preferably based on skin application of active agents. Accordingly, the active agents or compositions of the invention typically comprise one or several excipients suitable for topical application. Cosmetically acceptable vehicles are well known in the art and can be selected based on the end-use of the application. Common topical vehicles include hydrophobic vehicles, water-miscible vehicles, co solvents, structural matrix formers, suspending, jelling, or viscosity inducing agents, emulsifiers, and preservatives.

Suitable hydrophobic vehicles include:
- Hydrocarbons: Liquid petrolatum (mineral oil, liquid paraffin, paraffin oil), White petrolatum (petroleum jelly, Vaseline), Yellow petrolatum (petroleum jelly), Squalane (perhydrosqualene, spinacane),
- Silicones: Liquid polydimethylsiloxanes (dimethicone, silastic, medical grade silicone oil)
- Alcohols: Lauryl alcohols (1-dodecanol, dodecyl alcohols), Myristyl alcohols (tetradecanol, tetradecyl alcohols), Cetyl alcohols (hexadecanol, ethal, palmityl alcohols), Stearyl alcohols (stenol, cetosteryl alcohols), Oleyl alcohols (ocenol)
- Sterols; sterol esters: Lanolin (hydrous wool fat, lanum), Anhydrous lanolin (wool fat, anhydrous lanum, agnin), Semi synthetic lanolins
- Carboxylic acids: Lauric acid, Myristic acid, palmitic acid, stearic acid, oleic acid
- Esters; polyesters: Cholesterol esters (stearate), Ethylene glycol monoesters, Propylene glycol monoesters, Glyceryl monoesters, Glyceryl monostearate, Sorbitol monoesters, Sorbitain monoesters, Sorbitol diesters, Sorbitan polyesters (spans, arlacels), Glyceryl tristearate, Lard, Almond oil, Corn oil, Caster oil, Cottonseed oil, Olive oil,Soyabean oil,Hydrogenated oils, Sulfated oils, Isopropyl myristate, Isopropyl palmitate.
- Ethers; polyethers: Polyethylene-polypropylene glycols (pluronics)

Suitable water-miscible vehicles, co solvents are:
- Polyols; polyglycols: Propylene glycol (1,2-propanediol), Glycerin (glycerol), Liquid polyethylene glycol, Solid polyethylene glycol (hard macrogol, carbowax), 1,2,Phenols-hexanetriol, Sorbitol solution 70%
- Esters; polyesters: Polyoxyethylene sorbitain monoesters (stearate- tweens),Polyoxy ethylene sorbitan polyesters (tweens)
- Ethers; polyethers: Polyethylene glycol monocetyl ether (cetomacrogol 1000), Polyethylene-polypropylene glycols (pluronics)

Examples of preferred structural matrix formers include:
Hydrocarbons : White petrolatum (petroleum jelly, vaseline), Yellow petrolatum (petroleum jelly),Paraffin (paraffin wax, hard paraffin), Microcrystalline wax,Ceresin (mineral wax, purified ozokerite),
Silicones: Fumed silica (cab-O-sil),Bentonite (colloidal aluminum silicate),Veegum (colloidal magnesium aluminum silicate)
Polyols, polyglycols: Solid polyethylene glycol (hard macrogol, carbowax)
Alcohols: Cetyl alcohols (hexadecanol, ethal, palmityl alcohols), Stearyl alcohols (stenol, cetosteryl alcohols)
Sterols; sterol esters: Cholesterol (cholesterin), Lanolin (hydrous wool fat, lanum), Anhydrous lanolin (wool fat, anhydrous lanum, agnin), Semi synthetic lanolin's Carboxylic acids: Lauric acid, Myristic acid, palmitic acid, stearic acid, oleic acid Esters; polyesters: Bees wax, White bees wax (bleached bees wax), Carnauba wax, Myricin, Cholesterol esters (stearate), Polyoxyethylene sorbitain
Monoesters (stearate- tweens), Lard, Hydrogenated oils.

Suitable suspending, jelling, or viscosity inducing agents are:
Silicones: Fumed silica (cab-O-sil),Bentonite (colloidal aluminium silicate),Veegum (colloidal magnesium aluminium silicate)
Polycarboxylates; polysulfates; polysaccharides: Agar, Alginates, Carragen, Acacia, Tragacanth,,Methylcellulose, Carboxy methylcellulose, Hydroxy ethylcellulose, Carboxy vinyl polymer, gelatin, pectin, xanthan,polyacrylic acid.
Others: Ethanolamin, Triethanolamin.

Examples of Water-in-oil(w/o) emulsifiers include:
Sterols; sterol esters : Cholesterol (cholesterin),Lanolin (hydrous wool fat, lanum),
Anhydrous lanolin (wool fat, anhydrous lanum, agnin), Semi synthetic lanolins Carboxylic acids: Na+, K+, ethanolamin salts of Lauric acid, Myristic acid, palmitic acid, stearic acid, oleic acid.
Ethers; polyethers: Polyethylene-polypropylene glycols (pluronics)

Examples of Oil-in-water (o/w) emulsifiers are:
Esters;polyesters: Polyoxyethylene sorbitain monoesters (stearate- tweens), Polyoxy ethylene esters (stearate-polyethylene glycol,monoesters, Myrj), Polyoxy ethylene sorbitan polyesters (tweens)
Ethers; polyethers: Polyethylene glycol monocetyl ether (cetomacrogol 1000), Polyethylene-polypropylene glycols (pluronics)
Others: Sodium lauryl sulfate, Borax (sodium borate), Ethanolamine, Triethanolamine.

Suitable preservatives include:
Antimicrobial: Benzalkonium chloride, Benzoic acid, Benzyl alcohol, Bronopol, Chlorhexidine, Chlorocresol, Imidazolidinyl urea, Paraben esters, Phenol, Phenoxyethanol, Potassium sorbate, Sorbic acid
Antioxidants: a-Tocopherol, Ascorbic acid, Ascorbyl palmitate, Butylated hydroxyanisole, sodium ascorbate, sodium metabisulfite
Chelating agents, Citric acid, Edetic acid Buffer: Citric acid and salts, Phosphoric acid and salts, H3PO4 /NaH2PO4, Glycine, Acetic acid, Triethanolamine, Boric acid. Humectant: Glycerin (glycerol), propylene glycol (E 1520), glyceryltriacetate (E1518), sorbitol (E420), xylitol and maltitol(E965), polydextrose (E1200), quillaia (E999), lactic acid, urea, lithium Chloride.
Sequestering antioxidant: Citric acid and salts, Ethylenediaminetetraacetic acid
(Versene, EDTA)

The compositions may be formulated as solid compositions, such as a powder, aerosol or a plaster, as liquid compositions, such as a lotion, liniment, solution, emulsion, including micro-emulsions or nano-emulsions, suspension, or semi-solid compositions, such as a cream, gel, paste, or ointment.

The compositions may be applied topically, on the surface of the skin.

Alternatively, the compositions of the invention can be applied using a transdermal delivery system, such as liposomes and other lipidic vesicles, patch devices and technical textiles, etc. or any enhancement method. Several transdermal products are currently on the market, and are known by the skilled person in the art. Transdermal products can be meant for local dermal delivery, or for systemic delivery. Enhancement methods include chemical methods, physical methods and a prodrug approach.

Physical enhancement methods can also provide a greater magnitude of skin permeabilization. Such methods include the use of hypodermic needles, such as fillers. In order to exert a long-lasting effect, the HGP inhibitor can be applied using a procedure similar to those of injectable dermal fillers, for e.g., to inject the product directly into the hair follicle.
Several alternative physical skin enhancement methods may also be used such as jet injections, dermabrasion, thermal ablation, laser, microneedles, iontophoresis, electroporation, ultrasound and combinations of the above.
Electroporation- is based on the use of short (micro- to milli-second), high-voltage electrical pulses to create transient disruptions in the structure of the stratum corneum. Such disruptions, or pores, facilitate the transport of small and large molecules otherwise unable to permeate at all. Drug molecules are believed to be transported through skin by electrophoretic movement and diffusion. Examples proving *in vitro* effectiveness of electroporation include small molecules such as timolol, as well as larger molecules such as oligonucleotides, heparin and IgG antibodies (Creation of transdermal pathways for macromolecule transport by skin electroporation and a low toxicity, pathway-enlarging molecule. Bioelectrochem Bioenerg 1999;49(1):11-20. [PubMed: 10619443]).
Ultrasound skin enhancement (sonophoresis) is achieved by the use of high (MHz) or low (kHz) frequency devices, the latter showing greater percutaneous drug flux improvement. At low frequencies the ultrasound applied to the skin results in the production of cavitational bubbles, which by oscillation disrupt the stratum corneum structure. *In vitro* experiments have shown substantial increase in the skin permeability to small molecules such as estradiol, aldosterone or lidocaine, as well as macromolecules such as insulin, erythropoietin or γ-interferon. In 2004, the FDA approved an ultrasound system (SonoPrep®) to accelerate the action of lidocaine for local dermal anesthesia. The same system is intended to be used for delivering vaccines, insulin and antibiotics, and blood glucose monitoring in the future. This method does not cause pain or irritation when used within certain limitations.
Combinations of enhancement techniques have the potential of showing synergistic effects. Some combinations that have been studied include electroporation-ultrasound, electroporation-iontophoresis and microneedle-iontophoresis. The observed synergistic effects may be rationalized by different mechanisms by which enhancement methods work, for example electroporation disrupts stratum corneum barrier structure increasing its permeability to drugs, while iontophoresis provides additional driving force for diffusion of charged particles above that of just passive diffusion.

A prodrug approach may also be used for the composition of the invention to increase permeation across skin, chemical and enzymatic stability, as well as limited skin irritation potential. This approach is based on modifying the physicochemical properties of a parent drug such that the flux of the prodrug is increased over that of the parent molecule.

Nanoparticles could also be used for the delivery of the composition of the invention into the hair follicle. Nanoparticles are important carrier systems for the transport and storage into the skin of topically applied substances and compositions used in medicine and cosmetics. Interestingly, while in the last decades it was thought that topically applied permeated through the skin barrier (*Stratum Corneum*) by diffusion in the lipid layer surrounding the corneocytes, recent *in vivo* and *in vitro* investigations have found that the hair follicles also play a significant role in skin penetration, and represent and efficient reservoir for topically applied substances. Nanoparticles have been shown to play an important role in follicular penetration, while from nanoparticles obtained from TiO₂ frequently used in sunscreens, it is known that they do not pass the skin barrier, even if the size is in the region of 30 nm. It has been demonstrated that nanoparticles of 300 to 600 nm penetrate into the hair follicle, this size corresponding to the thickness of the cuticula of the hairs (Lademann & al 2008).

### Application

The method of the invention may comprise one or several applications of the compositions to the skin. Such repeated applications may be performed at various time intervals, such as for instance, once a day, once a week, once a month, or once every two months, for a period of time, which may be adjusted depending on the subject. Furthermore, the compositions may be applied directly on intact skin, or after pretreatment of the skin (e.g., by stripping, alcohol cleaning, moisturizing, etc.), or by massage.

In a further particular aspect, the invention resides in a method for retarding or limiting hair growth comprising applying to an area of the skin of a subject an effective amount of a composition as defined above.

In a further particular aspect, the invention resides in a method for increasing hair growth comprising applying to an area of the skin of a subject an effective amount of a composition as defined above.

The compositions may be applied alone.

Alternatively, the compositions of the invention may be used in combination with (e.g., before, simultaneously, or after) any other methods to remove hairs such as chemical, mechanical or physical depilation such as, for example, waxing, plucking or shaving, a medical or beauty device or method, such as lasers, LED, flashlights, light, radiofrequences, ultrasounds, electrotherapy, infra-reds, ionophoresis, heat, vapor, massage and aspiration devices, cryotherapy, etc. The invention may be used in any mammalian subject, typically in human subjects. The composition may be used on any part of the body where body hair needs to be removed, typically for aesthetical purposes, e.g. the face, legs, armpits, back, neck, chest, bikini, etc.

The invention may be used on any type of hair (e.g., any color, length, density, nature). Also, if appropriate, the treatment or composition may be adjusted depending on the type of hair or subject to provide best retarding effect.

### Immunization

Another aspect of the invention is to induce, in a subject in need thereof, antibodies against a HGP. According to this aspect, a subject is administered with an immunogenic composition comprising a HGP under conditions suitable to produce an immune response against the HGP. The subject will therefore produce antibodies that will permanently alter hair growth.

Preferably, the immunogen comprises a human immunogenic HGP or a fragment thereof. Typically, the HGP or fragment is coupled to a carrier to increase immunogenicity, or is combined with an adjuvant. Upon injection, the subject will produce an immune response sufficient to alter hair growth. The HGP is most preferably specific for body hair growth and not present or involved in hairs.

Such an aspect of the invention may be particularly suited in subjects having highly developed pilosity.

Further aspects and advantages of the invention will be disclosed in the following experimental section.

### EXAMPLES

### A - Production of an anti-HGP polyclonal antibody

Polyclonal antibodies were produced in New Zealand rabbits according to the method developed below.

### Protocols

### Immunization program

New Zealand rabbits were immunized by intradermous injections of the immunogen extracted from body hairs. Animal were injected at 2 weeks interval with 100 to 250 µg of urea extracted proteins (see below) in Complete Freund adjuvant ( 1^{st} immunization), and then in incomplete Freund adjuvant (2^{nd} to 5^{th} injections) during a 77 day protocol. Sera were collected 7 days after injections 3, 4 and 5 for investigation of antibody titer in an ELISA assay. 75 to 100 mL of sera was collected in total per each animal at completion of the protocol.

### Preparation of the immunogen

Hair bodies were collected in a Beauty Institute from voluntary donors that gave their approval to use their body hairs for research purposes. Body hair bulbs were individually collected from waxing strips under microscope and pooled. Proteins from body hair follicles were extracted essentially as described by Leung et al., Journal of Proteome Research, 2010. Briefly, body hair bulbs were solubilized by overnight incubation at 4°C PBS, pH 8.5, containing 6M urea and 5 µL protease inhibitor cocktail (SIGMA*FAST*^{™} Protease Inhibitor Tablets; ref S8820, Sigma-Aldrich) per mL . The supernatant containing extracted proteins was collected after centrifugation at 5,000 rpm during 15 mins for removal of non-solubilized proteins. Protein concentration was estimated by classical BSA protein assay. Aliquoted material was stored at -80°C.

### Results

### Preparation and characterization of the immunogen

400 hair individually cut out from the support provide 20 mg of total proteins. After solubilization in urea and centrifugation during 15 min at 5,000 rpm to remove insolubilized material, 0.6 mg of body hair proteins were obtained at a final concentration of 1 mg/mL.
Finally 6 to 7 µg of proteins were obtained from each body hair follicle individually cut out from the collecting piece.

Investigation of the protein profile of the immunogen was performed by sodium dodecyl sulfate polyacrylamide (SDS-PAGE) electrophoresis. In a typical analysis, a series of proteins was observed after Coomassie blue staining. Such a pattern was repeatedly obtained from several preparations. The major proteins were clearly depicted, showing no evidence of proteins degradation.

Under non-reducing conditions, a major component was observed at 250 kDa; it represents 35 to 45% of the total proteins entering the 4-20% polyacrylamide gel. Other proteins with apparent M.W. of 60, 57, 52, and 50 kDa were depicted after Coomassie blue staining. They represent approximately 27, 12, 3, and 22% of the stained proteins (mean of 2 experiments).

Under reducing conditions a similar pattern was observed. Of note, a protein at 45 kDa was observed; it represents roughly 10% of the proteins.

The HGP proteins identified on Coomassie blue stained gels were analyzed using commercial antibodies directed to hair follicle proteins using the Western blot technology (Towbin et al., 1979). Ten (10) µg of follicle proteins were electrophoretically transferred to Western blot nitrocellulose and probed with the specific antibodies: anti-ornithine decarboxylase, and anti-trichohyalin. Antibodies were incubated overnight at room temperature.

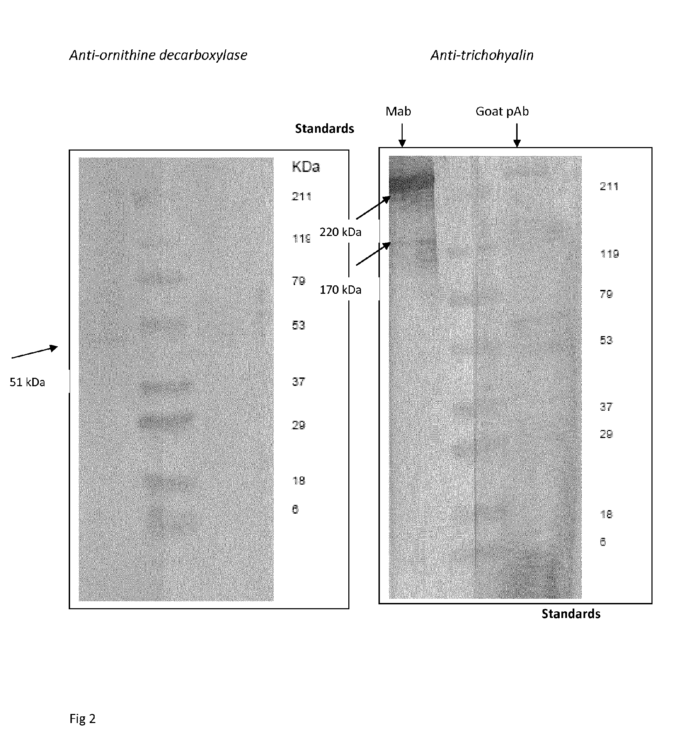

The results are presented Figure 2.

The results show that at least ornithine decarboxylase and trichohyalin are present in the immunogen. Indeed, the anti-ornithine decarboxylase antibody depicted a protein migrating with an apparent MW of 51 kDa, and the anti-trichohyalin monoclonal and polyclonal antibodies depicted bands at 220 and 170 kDa. These data fit with data given in the literature (data sheets from the products). They demonstrate the presence of Ornithine decarboxylase and Thrichohyalin in the follicle extract.

### Titers of the rabbit antibody obtained

Sera collected from the rabbits 7 days after injection of 100 to 200 µg of urea solubilized proteins were investigated by ELISA. Plates were coated with 100 µL of protein extracts at 5µg/mL in PBS pH 8.5. They were afterwards treated according to common procedure before applying the sera diluted in PBS.

Antibodies to body hair proteins could be demonstrated by ELISA. As an example, after 5 injections of limited amounts of material (100 to 200 µg per injection), antibodies with titers of 36,000 to 93,000 were obtained. The limit of detection of the antibodies (OD value < 0.2) was in the range of 500,000 to 1,000,000, demonstrating their high potency.

### Biochemical characterization of the anti-HGP polyclonal antibody

The antibodies obtained from immunized animals were tested and selected using the Western blot approach. The HGP proteins used as immunogen were electrophoretically transferred to Western blot nitrocellulose and probed with the rabbit sera diluted at 1/200 and 1/1,1000.

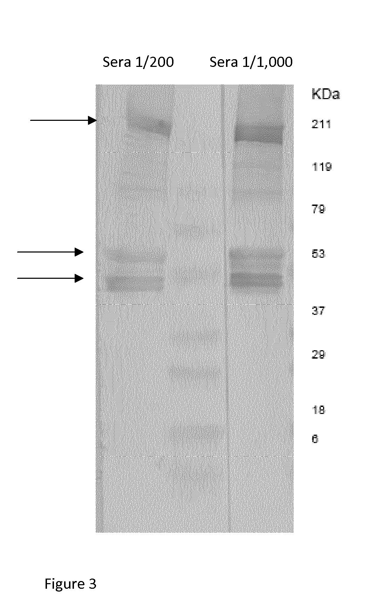

The results are presented Figure 3.

The results show that the polyclonal antibody (crude rabbit sera dilutes 1/200 or 1/1,1000) obtained recognizes proteins at 200 kDa and in the 40 to 50 kDa region. These bands correspond to ODC, trichohyalin, as well as spermine synthase (see also Fig. 2) confirming that the polyclonal antibody hereby produced binds major HGP proteins.

### Purification of the polyclonal antibody from collected sera

Antibodies from the animal sera were purified by affinity chromatography on Protein A-Sepharose according to commonly used techniques. Seven (7) to 8 mg of total Igs were obtained per mL of sera (pool of sera collected 7 days after the third immunization.).

### Antibody specificity

Non-specific antibodies are depleted from the preparation by adsorption of the crude extract of antibodies obtained after Protein-A chromatography on reconstituted human skin or epithelial cells.

### B - Anti-HGP antibodies inhibit hair growth

Anti-HGP antibodies were produced (see example A) or obtained from commercially available sources, and investigated for their ability to inhibit body hair growth ex vivo using human skin explants.

The activity of the antibodies was assessed based on their effect on the morphology and growth of the body hair.

The activity of keratinase A (Sigma) was investigated in parallel. Eflornithin (Calbiochem) was used as a reference.

### Selected antibodies

The selected antibodies are listed in the following table II.

**Table II**

| **Antibodies** | **Provider** | **Origin** | **Immunogen** |
|---|---|---|---|
| **Anti-ODC** | | | |
| Anti-Ornithine Decarboxylase antibody [1 G6] (ab119072) | Abcam | Mouse (monoclonal) | Recombinant full length Human Ornithine Decarboxylase produced in HEK293T cells |
| Anti-Ornithine Decarboxylase antibody (ab97395) | Abcam | Rabbit | Recombinant protein fragment containing a sequence corresponding to a region within amino acids 180 and 433 of Human Ornithine Decarboxylase |
| Anti-SLC25A21 antibody (ab74106) | Abcam | Rabbit | synthesized peptide derived from internal of human SLC25A21 |
| Anti-Ornithine Decarboxylase antibody [EPR5724] (ab126590) | Abcam | Rabbit | Synthetic peptide, corresponding to residues in Human Ornithine Decarboxylase |
| | | | |

| **Anti-Trichohyalin** | | | |
|---|---|---|---|
| Anti-Trichohyalin antibody [AE15] (ab58755) | Abcam | Mouse (monoclonal) | Urea-mercaptoethanol extract of human hair follicles |
| Anti-TCHHL1 antibody (ab82141) | Abcam | Goat | Synthetic peptide QALEDKQGHPQRE, corresponding to internal sequence amino acids 878 - 891 of |
| Trichohyalin (N-16) Anticorps sc-47517 | Santa Cruz | Goat | Peptide mapping near the N-terminus of Trichohyalin of human origin |
| Trichohyalin (K-16) Anticorps sc-47515 | Santa Cruz | Goat | peptide mapping at the C-terminus of Trichohyalin of human origin |
| | | | |

| **Anti-K16** | | | |
|---|---|---|---|
| Anti-STK16 antibody - Aminoterminal end (ab37975) | Abcam | Rabbit | KLH conjugated synthetic peptide, selected from the N-terminal sequence of human STK16 within aa 1-100 |
| Anti-STK16 antibody (ab85638) | Abcam | Rabbit | Synthetic peptide corresponding to a region within the internal sequence amino acids 215-264-(TDVWSLGCVLYAMMFGEGPYDMVFQKGDSVALA VQNQLS IPQSPRHSSAL) of Human STK16 |
| WIF-1 (K-16) sc-30791 | Santa Cruz | Rabbit | peptide mapping near the C-terminus of WIF-1 of human origin |
| | | | |

| **Anti-Spermine synthase** | | | |
|---|---|---|---|
| Anti-Spermine synthase antibody (ab101458) | Abcam | Rabbit | Recombinant fragment corresponding to a region within amino acids 144-336 of Human Spermine synthase |
| Anti-Spermine synthase antibody (ab107536) | Abcam | Rabbit | Recombinant protein of Human Spermine synthase amino acids 1-299 |
| | | | |

| **Anti-Elastase antibody** | | | |
|---|---|---|---|
| Anti-Elastase antibody (ab1876) | Abcam | Rabbit | Elastase [Porcine Pancreas] |
| Anti-Elastase antibody (ab30525) | Abcam | Rabbit | Full length native protein (purified) (Human) |

### Protocols

### Preparation of human explants

40 explants of a diameter of about 11 mm (± 1 mm) were obtained by abdominoplasty from 2 men aged 23, and 19 years. The explants were placed in survival in the BEM (BIO-EC's Explants Medium) at 37° C in a humid atmosphere enriched with 5% CO2.

### Application of the products

The treatment was performed at D0, D1, D4, D6 and D8 by topical application of 5 µl of diluted antibody used at final concentrations of 30 to 100 µg/mL in PBS. Eflornithin was used at a concentration of 100 mg/mL and keratinase at 20 mg/mL. A negative control experiment was performed using PBS only.

### Samples

At D0, T0 lot explants were removed and cut in half. Half was fixed in buffered formalin and the other half was frozen at -80 ° C.
A D8, 2 explants from each batch were taken and treated in the same manner.

### Histological processing

After 24 hours of fixation in buffered formalin, the samples were dried and impregnated with paraffin using an automatic dehydration Leica TP 1010 according to the procedure MO-H-149, according to our procedure using a station coating Leica EG 1160. Sections of 5 microns were performed, using a microtome kind Minot, Leica RM 2125 and mounted on silanized glass slides Superfrost ® plus.
Microscopic observations were performed by microscopy optical transmission. The shots were taken with a Leica Orthoplan DMLB or with a digital camera Olympus DP72 driven software acquisition and archiving Olympus CellD.
The morphology was examined on paraffin sections stained with Masson's trichrome Goldner variant.

### Measurement of hair growth

At D0 and D8, the explants were photographed and the length of each individually hair was measured using the measuring module Olympus software CellD.

### Results

The results are summarized in the Table below.

**Table**

| **ID** | **Designation** | **Reference** | **Upper part** | **Sebaceous gland** |
|---|---|---|---|---|
| A11 | Anti-Ornithine Decarboxylase [1G6] | ab119072 | +++ | +++ |
| A12 | Anti-Ornithine Decarboxylase | ab97395 | +++ | ++++ |
| A 13 | Anti-SLC25A21 | ab74106 | +++ | |
| A14 | Anti-Ornithine Decarboxylase [EPR5724] | ab126590 | +++ | +++ |
| A21 | Anti-Trichohyalin [AE15] | ab58755 | +++ | |
| A22 | Anti-TCHHL1 | ab82141 | ++ | |
| A23 | Anti-Trichohyalin (N-16) | sc-47517 | + | +++ |
| A24 | Anti-Trichohyalin (K-16) | sc-47515 | +++ | +++ |
| A31 | Anti-STK16 - Aminoterminal end | ab37975 | +++ | +++ |
| A32 | Anti-STK16 antibody | ab85628 | ++++ | ++++ |
| A33 | Anti-WIF-1 (K-16) | sc-30791 | +++ | ++++ |
| A51 | Anti-Spermine synthase | ab101458 | +++ | |
| A52 | Anti-Spermine synthase | ab107536 | + | ++++ |
| A61 | Anti-Elastase | ab1876 | ++ | ++ |
| A62 | Anti-Elastase | ab 30525 | + | ++++ |
| R1 | Eflornithine at 100 mg/ml. - Batch D00127044 | 288500 | ++++ | ++++ |
| Ker | Keratinase A - Batch SLBC5798V | K4519-500UN | +++ | +++ |

| | | | | |
|---|---|---|---|---|
| Quotation of morphological alterations from - (no alteration), + (weak), ++ (moderate), +++ (significant), ++++ (very significant), +++++ (strong). | | | | |

**Definition of a Pycnotic nucleus:** condensation of the cell nuclear content ; the nucleus retracts and becomes hypersensitive to coloration, this indicating a nucleus degeneration leading to cellular necrosis.

### General Morphology of the Hair Follicle

Whatever the sample examined, the morphology of the epidermis was not altered.

### Control group at D0 (T0)

Upper part of the hair follicle, near the infundibulum : the epithelial sheet exhibits no cellular alteration, a very thick *Stratum Corneum,* well foliated, with a clearly keratinized base, can be observed.
Midsection, at the sebaceous glands level: the inner and outer sheath exhibit a good morphology without cell damage.
Lower part of the hair follicle : the morphology of the inner and outer root sheats is good without cellular alterations.

### Control group at D8 (T8)

Upper part of the hair follicle, near the infundibulum: The part near the infundibulum could not be observed.
Midsection, at the sebaceous glands level: the inner and outer sheath exhibit a good morphology without cell damage.
Lower part of the hair follicle: the morphology of the inner and outer root sheats is good with very slight cellular alterations (cells with pycnotic nucleus)

### Group treated with the reference product - Eflornithine at 100 mg/mL at D8

The morphology of the epidermis is slightly altered with a slight spongiosis.
- Upper part, near the infundibulum: The internal and external epithelial sheath show sharp cellular alterations characterized by the presence of numerous nucleated cells significantly pycnotic. The upper limit of the alteration is very clear.
- Midsection, sebaceous glands: At the sebaceous glands level, inner and outer root sheaths show clear cellular alterations characterized by the presence of numerous nucleated cells significantly pycnotic. The region below the outer root sheath is the most affected.

- Lower part of the hair follicle: the inner and outer root sheath show very slight cell alterations with some cells slightly pycnotic.
- Bulb: The morphology of the bulb in the anagen phase is comparable to that observed in the untreated group D8. The morphology of the cells of the dermal papilla is good.

### Groups treated with antibodies at D8 (TA11 to TA62)

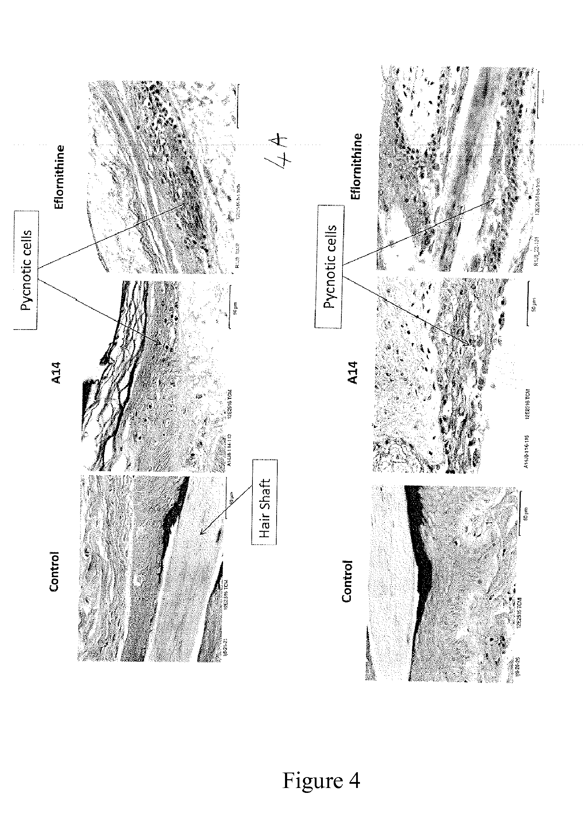

Whatever the group and in general, the morphology of the epidermis is not altered.
For the entire series of tested antibodies, their application induces alterations of the inner and outer root sheats of the hair follicle (as shown by cells with a pycnotic nucleus), in the upper region near the infundibulum.
In the sebaceous glands region, alterations also appear, with the formation of neokeratinocytes in some of the samples.
In the lower part of the hair follicle, the epithelial sheets exhibit a rather good morphology, the sebaceous gland probably marking the limit zone for penetration of the product, in the conditions of the test (see Figure 4).

The results demonstrate that topically applied antibodies alter the hair follicle structure and therefore impair hair growth. The results obtained with the tested antibodies are very similar to the results obtained with reference product (eflornithine), without any optimization of the conditions of application or of the formulation, and at low antibody doses.

### C - Anti-HGP antibodies retard the growth of hair follicles

Hair growth is measured using standard morphometric studies on isolated human hair follicles maintained in vitro (Philpott, 1999). Hair follicles are micro-dissected in a human scalp and maintained in a culture medium. Hair follicles in anagen phase are selected for the study. Each tested group is composed of 10-12 hair follicles. Hair follicles are photographed at D0, D4,D6 and D8 and their length is compared.
Control Group 0 : no application
Control Group E : application of eflornithine 10 µmol ; 2h00 at 37°C
Control Group M : application of Minoxidil 2% 10 µmol ; 2h00 at 37°C
Test groups A11, A13, A23, A24, A33 and AP1 : application of 30 mL of composition of the invention including the corresponding antibodies ; 2h00 at 37°C
Hair growth in test groups A11 to AP1 show significant hair growth retardation, when compared to Control group E. Both show significant hair growth retardation when compared to Control Group 0 and to Control Group M.
While chemical compounds, such as eflornithine, have short half-lives (30 mn to 1 hour), antibodies can remain active for several days, thereby further sustaining the growth retardation effect.

In a further experiment, a keratinase, such as KerA from Bacillus subtilis, is applied to the hair follicles during 1h00 at 20 mg/mL, and then washed. The compositions of the invention are subsequently applied to the hair follicles. The keratinase alters the epithelial sheets of the hair, thus enabling the delivery of the antibodies of the invention into the hair follicle.

Globally, the experiments demonstrate a significant effect of antibodies directed to ornithine decarboxylase, trichohyalin, and spermine synthase.
These results show that topic antibodies can reach the hair follicle and exert a biological effect on hair growth. The antibodies significantly altered the structure and/or growth of body hairs, depending on the target HGP.

### D - Formulations of anti-HGP antibodies

The following suitable lotions and creams comprising an antibody of the invention are prepared by conventional methods:

### Hair growth retardation and depilatory Lotions

**The following lotions are produced :**

| Concentrate | % w/w |
|---|---|
| Anti-HGP antibody | 2.5 |
| Ker A | 3.5 |
| Cetyl alcohol | 4.0 |
| Mineral Oil | 4.0 |
| Isopropyl Myristate | 1.0 |
| Dimethicone | 1.0 |
| Lanolin alcohol | 0.5 |
| Glycerol monostearate | 1.0 |
| Sodium lactate | 1.4 |
| Dimethyl diammonium chloride | 2.0 |
| Propylene glycol | 3.0 |
| Methyl paraben | 0.2 |
| Propyl paraben | 0.1 |
| Titanium dioxide | 0.1 |
| Perfume | 0.1 |
| Water | 75.6 |

**Lotion 2 :**

| Concentrate | %w/w |
|---|---|
| Anti-HGP antibody | 2.3 |
| Thioglycolate | 3.0 |
| Ker A | 3.5 |
| Stearic acid | 8.0 |
| Cetyl alcohol | 3.0 |
| Propyl parasept | 0.1 |
| Glycerin | 4.0 |
| Methyl paraben | 0.1 |
| Perfume | 1.0 |
| Water | 75 |

## Claims

1. A composition for use for modifying hair growth in a subject, comprising an antibody, or an agonist thereof, that binds a hair growth molecule.

2. The composition for use of claim 1, wherein the antibody is a polyclonal or a monoclonal antibody, or a fragment or derivative thereof having substantially the same antigen specificity, preferably selected from Fab, ScFv, CDR, human or humanized antibody.

3. The composition for use of claim 1 or 2, wherein the hair growth molecule is a protein of the polyamine pathway, preferably selected from ODC, spermidine synthase, and spermine synthase.

4. The composition for use of claim 1 or 2, wherein the hair growth molecule is a hair follicle protein.

5. The composition for use of claim 4, wherein the hair follicle protein is a keratin or KFAP protein, preferably selected from Trichohyalin, keratin K16, and filaggrin.

6. The composition for use of any one of the preceding claims, wherein the composition comprises a combination of at least two antibodies, or agonists thereof, which bind a distinct hair growth molecule or distinct epitopes of a same hair growth molecule.

7. The composition for use of any one of the preceding claims, wherein the composition comprises at least one antibody, or an agonist thereof, that binds ODC and at least one antibody, or an agonist thereof, that binds trichohyalin.

8. The composition for use of any one of the preceding claims for retarding hair growth, wherein the antibody, or the agonist thereof, inhibits the Hair Growth Protein.

9. The composition for use of any one of the preceding claims, wherein the composition further comprises an enhancer.

10. The composition for use of claim 9, wherein the enhancer modifies hair growth or structure and/or enhances the activity of the antibody, preferably its delivery into the hair follicle.

11. The composition for use of claim 9 or 10, wherein the enhancer is selected from a depilatory agent, an anti-angiogenic agent, an anti-inflammatory agent, or a hair stimulating agent.

12. The composition for use of anyone of claims 9 to 11, wherein the enhancer is a keratinolytic protease, preferably a keratinase.

13. The composition for use of anyone of claims 9 to 11, wherein the enhancer is a chemical compound that alters hair growth, preferably selected from anti-ODC agents, and thioglycolate compounds.

14. The composition for use of anyone of the preceding claims, wherein the composition further comprises at least one compatible excipient or carrier, preferably a nanoparticle or a lipophilic vesicle.

15. The composition for use of any one of the preceding claims, wherein the composition is a solid composition, such as a powder, aerosol or a plaster, a liquid composition, such as a lotion, liniment, solution, liposome, emulsion, including micro-emulsion or nano-emulsion, nanoparticle, suspension, or a semi-solid composition, such as a cream, gel, paste, or an ointment.

16. The composition for use of anyone of the preceding claims, wherein the composition comprises:
(i) an antibody, or an agonist thereof, that inhibits an activity of a hair growth protein of the polyamine pathway or a keratin or KFAP protein;
(ii) an enhancer selected from a depilatory agent, an anti-angiogenic agent, or an anti-inflammatory agent; and
(iii) an excipient or carrier.

17. The composition for use of any one of the preceding claims, wherein the composition is administered by dermal application.

18. The composition for use of claim 17, wherein the composition is applied topically on an area of the skin of the subject.

19. The composition for use of claim 18, wherein the composition is injected in or below the skin of the subject.

20. The composition for use of any one of the preceding claims, wherein the composition is applied repeatedly.

21. The composition for use of any one of the preceding claims, wherein the composition is applied before or after chemical, mechanical or physical depilation or shaving.

22. The composition for use of any one of the preceding claims, wherein the composition is applied with, or formulated in, a device, a patch device or a technical textile.

23. The composition for use of any one of the preceding claims, wherein the composition comprises between about 1 µg/mL to about 50mg/mL antibody.

24. A topical composition comprising an antibody, or an agonist thereof, that binds a hair growth molecule, and a topical excipient.

25. A composition comprising (i) an antibody, or an agonist thereof, that binds a hair growth protein, (ii) an enhancer, and (iii) a suitable excipient or carrier.

26. The composition of claim 25, wherein the antibody or agonist thereof is an antibody or an agonist thereof that reacts with a hair growth protein of the polyamine pathway or reacts with a keratin or KFAP protein, preferably against a protein selected from ODC, spermidine synthase, spermine synthase, trichohyalin, keratin or filaggrin.

27. A composition comprising a trichohyalin inhibitor for use to modify hair growth.

28. A composition comprising a compound selected from N-(4'-Pyridoxyl)-Ornithine(BOC)-Ome, 4-amidinoindan-1-one-2'-amidinhydrazone, S-adenosyl-1,12-diamino-3-thio-9-azadodecane, or N1,N11-Diethylnorspermine tetrahydrochloride, for use to alter hair growth.

29. The composition of any one of claims 24 to 28, which is a solid composition, such as a powder, aerosol or a plaster, a liquid composition, such as a lotion, liniment, solution, liposome, emulsion, including micro-emulsion or nano-emulsion, suspension, nanoparticle or a semi-solid composition, such as a cream, gel, paste, or ointment.
